(19) Europäisches Patentamt
European Patent Office
Office européen des brevets

(11) **EP 1 867 346 A1**

(12) **EUROPEAN PATENT APPLICATION**

(43) Date of publication:
**19.12.2007 Bulletin 2007/51**

(51) Int Cl.:
**A61L 9/015** (2006.01)    **F24F 3/16** (2006.01)
**A61L 9/22** (2006.01)

(21) Application number: **06115390.4**

(22) Date of filing: **13.06.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **askair Technologies AG**
**8022 Zürich (CH)**

(72) Inventors:
- **Kasper, Andreas U., Dr.**
  **8942 Oberrieden (CH)**
- **Bishop, Ducan Aleck**
  **Huntingdon, Cambridgeshire PE28 4EA (GB)**

- **Jarvis, Jonathan William**
  **Cambridgeshire CB4 5JU (GB)**
- **Lloyd-Lucas, Dominic**
  **Cambridgeshire CB4 8SW (GB)**
- **Thomas, Cornelia Juliane Felicitas**
  **Cambridge CB2 2EQ (GB)**
- **Toutoungi, Danielle Emma**
  **Cambridge CB1 3AW (GB)**
- **Wrench, Nathan James**
  **Cambridgeshire CB5 0JX (GB)**

(74) Representative: **Hepp, Dieter et al**
**Hepp, Wenger & Ryffel AG,**
**Friedtalweg 5**
**9500 Wil (CH)**

(54) **Air treatment device and method of treating a gaseous medium**

(57)    The invention discloses the use of a corona plasma electrode, preferably the reference electrode as a recipient for precipitation of contaminants of a gaseous medium conducted through a flow-through air treatment device (1), said air treatment device (1) comprising a downstream ozone mitigation device (4). The duration of exposure of said contaminant to reactive oxygen species, especially ozone ($O_3$), is thereby prolonged up to or exceeding an effective value necessary for decontamination.

Fig. 1:

EP 1 867 346 A1

**Description**

The present invention relates to the field of air treatment devices, more particularly to air treatment devices for the decontamination of air, especially in HVAC systems.

Today, the necessity of decontamination of air is almost omnipresent. For example in the heating, ventilation and air conditioning (HVAC) industry, it is an outstanding challenge to continuously provide healthy, non-malodorous air - especially in times of potential biological and/or chemical attacks that could be carried out using the rapid distribution of contaminants via HVAC systems.

Various approaches are currently known for decontamination of air:

Electrostatic filters are widely used, that are located downstream of an ioniser. Dust particles are firstly charged by the ioniser, and subsequently attracted by the oppositely charged filter. Evidently, such devices are only effective for ionisable particles, and do not provide any further destructive effect on precipitated particles which thus may remain in the filter in an active state (e.g. in the case of hazardous microorganisms).

Moreover, various air treatment devices are known that use the destructive effect of ozone (e.g. due to ozonolysis). Ozone is commonly generated e.g. by means of a corona discharge or UV radiation outside of the flow-path of the medium to be treated (air), and the ozone is subsequently fed into the air stream. Such devices are *inter alia* known from GB 2 413 377 A and WO 03/028773 A1. At least one apparent deficiency of such systems is that decontamination wholly depends on ozone (or other reactive oxygen species). However, not necessarily all contaminants of a medium to be treated are susceptible to and destructible by ozone. Next, the duration of exposure is often not sufficiently long for some contaminants to be destructed.

Moreover, from WO 00/33945 a combination of a low-temperature plasma and electrostatic filtration is used in treating combustion gases of wood and petroleum products. A thin wire is located in the centre of an exhaust channel, extending in the longitudinal direction. The inner walls of the exhaust channels are electrically conducting, too. Thereby, a non-thermal plasma can be generated between the wire and the exhaust channel's wall. It is stated that a large percentage of particles will be oxidized before they are discharged from the channel; particles which are too large to be oxidized are however only attracted by the grounded channel wall. Thus, the efficiency of this device mainly depends on the size of the particles, and e.g. bio-contaminants such as microorganisms might only be attracted like in an electrostatic filter, but not effectively destroyed. This, in fact, is not an issue in combustion channels, but can not be accepted in the above-mentioned HVAC applications.

It is thus an object of the present invention to overcome at least some of the drawbacks of the prior art, especially to provide an air treatment device that is effective and reliable in the treatment of a vast variety of contaminants in air, and which can be easily installed and is easily to be maintained.

This object is surprisingly met by an air treatment device and a method of treating a gaseous medium, according to the independent claims.

The flow-through air treatment device according to the present invention comprises:

i) an inlet for a gaseous medium to be treated,
ii) an air treatment zone, comprising a corona discharge device located in said air treatment zone, which corona discharge device in use creates ozone ($O_3$) at a concentration of at least 1 ppm, preferably about 1 ppm to about 50 ppm, most preferably about 2 ppm to about 20 ppm;
iii) a downstream ozone mitigation device, allowing for a reduction of the ozone ($O_3$) concentration to below about 0.100 ppm, preferably below 0.050 ppm, most preferably below 0.030 ppm;
iv) an outlet for the treated gaseous medium.

It has now been found that by combining the destructive effect of an effective concentration of ozone with the electrostatic precipitation of contaminants in an environment comprising said effective concentration of ozone surprisingly enhances the efficiency of air treatment devices significantly.

The corona discharge device serves various aspects: Firstly, it is operated under such operating conditions that the above-mentioned, effective concentrations of ozone are generated. Secondly, the electrode(s) of the corona discharge device serve(s) as a recipient for the precipitation of (due to the ionising effect the plasma) ionised contaminants, thereby significantly prolonging the duration of exposure of the contaminants to the above-mentioned concentrations of ozone. Moreover, the precipitated contaminants are subjected to a high electric field on the electrode, additionally aiding in destruction. Thirdly, the ozone is created directly at its desired place of action, thereby overcoming the drawback of the prior art devices which feed ozone from outside into the flow-path, thus accepting a decrease in the concentration of ozone and other reactive oxygen species, when compared to the originally generated quantity of ozone.

As used herein, the term "contaminant" shall be understood as to especially comprise microorganisms such as bacteria, algae, fungi, etc., but also phages, viruses, etc., as well as gaseous contaminants, e.g. malodorous gases (e.g. volatile organic compounds, VOC) or other gases that might be noxious, even though not necessarily malodorous, e.g. pesticides, chemical weapons agents, etc.

According to preferred embodiments of the present invention, the corona discharge device generates an electric field of about $10 \times 10^6$ V/m to about $20 \times 10^6$ V/m. This range has shown to be a working compromise of high field intensity (generally wanted) and the risk of a spark-over (to be avoided).

Preferably, an energy density of about 400 J/m$^3$ to about 4,000 J/m$^3$ is created in the treatment zone, albeit higher energy densities may be used; with a positive corona discharge device, the minimum energy density is preferably adjusted to $\geq 800$ J/m$^3$. These ranges provide for a sufficient kill rate for almost all currently tested microorganisms.

The current density on the surface area of a field electrode is preferably within the range of about 0.4 A/m$^2$ to about 4.0 A/m$^2$; with a positive corona discharge device, the current density is preferably adjusted to $\geq 0.9$ A/m$^2$. These ranges proved sufficient for a sufficient kill rate for almost all currently tested microorganisms, especially given a flow rate of about 2.5 m/s.

According to yet another preferred embodiment, the corona discharge device allows for establishing a positive corona plasma. The field electrode (the highly curved electrode, e.g. a wire) is positively charged, the reference electrode (comparatively flat) is connected to ground. The physics of positive and negative coronas are strikingly different. Although negative coronas produce, in general, more ozone than the corresponding positive corona analogue, the positive corona is preferred in the present invention. Firstly, the negative corona discharge is much more destructive to the field electrode, thus leading to a reduced lifetime. Next, the positive corona yet is sufficiently efficient in creating the above-mentioned ozone concentration. Moreover, the electrons generated in a positive corona are presumably of higher energy, because they are concentrated close to the field electrode, i.e. in a region of high-potential gradient. Whereas these higher-energy electrons are in fact not necessary for the production of ozone, they appear to aid in processes that also contribute to the decontamination of the gaseous medium and which require a larger activation energy than achievable by negative corona.

The corona discharge device may advantageously be supplied with a pulsed DC. Preferably, the pulsed current however does not fall below a current which is necessary for establishing a corona discharge. When the DC is pulsed in such a way that even in the valleys of the current curve the potential is kept sufficiently high for a corona discharge, a pulse could serve to efficiently "clean" the field electrode from contaminants such as e.g. positively negatively charged contaminants that are deposited thereon and thus might otherwise hamper a continuous, uniform plasma formation.

Further preferred embodiments of the device comprise a suction device, preferably a fan or a pump, which is arranged downstream of the ozone mitigation device. Owing to the suction device located at or nearby the outlet of the air treatment device, the medium to be treated is conducted through the air treatment device by a moderate vacuum. This offers the advantage that contaminants and/or ozone (or other reactive oxygen species) may not significantly leak from the air treatment device even in the case of a leakage in the device. In contrast, if the medium to be treated would be pushed through the device, contaminants and/or ozone (or other reactive oxygen species) could significantly spoil the outer environment by passing through such a leakage. Preferably, the suction device is operable in such a way to allow for the gaseous medium being conducted through the treatment zone at a flow rate of about 0.5 m/s to about 16 m/s, preferably about 0.5 m/s to about 5 m/s, most preferably about 2 to about 3 m/s. It is to be understood that depending on the downstream ozone mitigation device, the upper limit of the flow rate through the whole device might in practice be lower than the above-mentioned 2-3 m/s (e.g., with some catalytic mitigators, about 0.5-1.0 m/s are the upper limit). The ideal flowrate through the whole device can however be easily determined by the person of routine skill in the art.

As outlined above, contaminants are precipitated onto the electrode(s) of the corona discharge device. It could be shown that it is especially advantageous if an air treatment device especially as outlined above comprises a corona discharge device, wherein an electrode, preferably the reference electrode of said corona discharge device is made of or preferably essentially comprises a material with cytotoxic effect. Suitable materials, albeit perfectly known to the person of routine skill in the art as such, have not yet been used as an electrode of a corona discharge device, to the best of applicant's knowledge. Preferably, the material is selected from the group consisting of copper, silver, or combinations or alloys thereof. Alternatively or additionally, the electrode may include a coating with a cytotoxic effect. Substances suitable for such coatings are known to the person of routine skill in the art and may e.g. include copper or silver ions, or common antimicrobial materials based on organic acids and their salts, such as Potassium Sorbate. Especially microorganisms can be more efficiently destroyed when they are precipitated onto an electrode made of or comprising and/or coated with a cytotoxic material, when compared to an electrode made of e.g. stainless steel.

Most preferably, at flow rates of about 0.5 m/s to about 16 m/s, preferably about 0.5 m/s to about 5 m/s, most preferably about 2 to about 3 m/s, the corona discharge device is designed in such a way to allow for a transit time of the gaseous medium through the corona discharge device of about 30 ms to about 200 ms, preferably about 40 ms to about 175 ms, most preferably about 50 ms to about 150 ms. The transit time is to be understood as the time that a gaseous medium would need for passing the corona discharge device without any side-effects such as precipitation of contaminants to occur.

As outlined above, the corona discharge device is operable in such a way to allow for a precipitation of contaminants from the gaseous medium on an electrode, preferably the reference electrode. Thuslike, the duration of exposure of (precipitated) contaminants to ozone (and other reactive oxygen species) may greatly exceed the transit time as defined above. A duration of exposure of $\geq$ 15 min was easily achieved when the above-mentioned settings were applied, having proved to be sufficient for killing even relatively hardy species such as e.g. yeast.

According to a first preferred geometry of the device according to the invention, the treatment zone comprises at least one, preferably a multitude of corona discharge device(s), comprising an outer reference electrode of tube-like geometry as a flow-through passage for said gaseous medium to be treated, said reference electrode surrounding an inner field electrode of wire-like geometry. Preferably, the field electrode(s) is/are arranged essentially in parallel with the direction of the flow of the gaseous medium. This geometry is easy to apply and provides for an ideal arrangement of the electrodes with respect to their distance from each other. However, with large amounts of a medium to be treated, several tube-like electrodes would have to be used beside each other. The remaining space between these tubes would have to be sealed from the flow, thereby decreasing the effective cross-sectional area of the device: A significant part of the cross-sectional area could thus not be used for the flow-through of the medium to be treated.

According to yet another preferred geometry of the device according to the invention, the treatment zone thus comprises at least one, preferably a multitude of corona discharge device(s), comprising at least one reference electrode of plate-like geometry, and a field electrode of wire-like geometry, preferably two plate-like reference electrodes arranged on opposite sides of the wire-like field electrode. According to this embodiment, the whole cross-sectional area can be used for the flow of the medium to be treated. Albeit the surrounding of the field electrodes is not as homogeneous as compared to the tube-like geometry, a stable plasma can be generated, however. The field electrode(s) is/are preferably arranged essentially perpendicular to the direction of the flow of the gaseous medium. Several field electrodes may then be arranged successively in the direction of the flow. Thus, the medium to be treated is passed through a multitude of plasma "curtains". The plate-like electrode(s) is/are preferably the wall(s) of a flow-through passage for said gaseous medium to be treated. Of course, a multitude of such treatment zones of plate/wire geometry, as outlined above, can be stacked one upon the other and/or beside each other, thus allowing for an easy scale-up of the cross sectional area of the flow path, e.g. when higher flow rates are to be used.

As outlined above, the efficiency of the air treatment device *inter alia* depends on the effective ozone concentration and the flow rate of the medium to be treated. Thus, the air treatment device preferably comprises a flow-meter and/or an ozone ($O_3$) sensor, within the treatment zone and/or at the outlet. An ozone sensor at the outlet is used to assure that the concentration of remaining ozone is kept below a predefined threshold. If such a threshold value is exceeded, the air treatment device can e.g. be automatically shut down, or operated at reduced power. An ozone sensor in the treatment zone is used to assure that the concentration of ozone is kept in the above-mentioned concentration ranges which proved to be effective in decontamination. A flow-meter may advantageously be used in order to control the flow rate in the device in the above-mentioned preferred ranges. Moreover, flow-meters upstream (or directly at the inlet of the device) and downstream of the device (or directly at the outlet of the device) may be employed in order to assure that any blockage of the air treatment device can easily be detected and the appropriate measures being triggered immediately (e.g. an automatic shutdown).

As outlined above, the air treatment device according to the invention uses ozone in relatively high concentrations, which is potentially hazardous to human health. Thus, the ozone mitigation device needs to be reliably effective, but also provide for a comparably low back-pressure in order to allow for common flow-rates of HVAC systems to pass. Preferred ozone mitigation devices thus comprise a preferably disposable catalytic ozone mitigator, preferably comprising a zeolith with an $SiO_2/Al_2O_3$ ratio of not less than 15, and activated manganese dioxide ($MnO_2$). These compositions are preferably provided on a paper support of high porosity, thus allowing for sufficient flow-rates. Preferably, a disposable particle filter is arranged upstream of the ozone mitigation device, in order to protect the ozone mitigation device from particle deposition that otherwise might hamper the destruction of ozone. Of course, the invention is not to be limited to the above-mentioned ozone mitigation device; other ozone mitigators are perfectly known to the person of routine skill in the art, e.g. UV radiation of a wavelength resulting in ozone breakdown, or mitigators comprised of activated carbon. Activated carbon is widely-used in air-treatment applications, especially for the removal of odours and VOCs, but suffers from the disadvantages of a short operating life-time and a relatively large volume requirement compared to the catalytic-type ozone mitigator. Activated carbon filters rely on adsorption / absorption and ion-exchange mechanisms, so quickly become exhausted and ineffective when ozone levels are high, as in the present invention. They therefore require regular checking, and replacement when exhausted. Moreover, activated carbon filters might theoretically catch fire, especially in view of the high voltage supply nearby, as a potential source of ignition. Catalytic mitigators represent less of such fire hazard and are thus preferred.

The invention further relates to a corresponding method of treating a gaseous medium, comprising the steps of:

- preferably continuously conducting said gaseous medium through a treatment zone, said treatment zone comprising at least one preferably positive corona plasma device creating ozone ($O_3$) at a concentration of at least 1 ppm, preferably about 1 ppm to about 50 ppm, most preferably about 2 ppm to about 20 ppm;
- precipitating contaminants from the gaseous medium on an electrode, preferably the reference electrode(s) of said corona plasma device;

- conducting said gaseous medium through a downstream ozone mitigation device, allowing for a reduction of the ozone ($O_3$) concentration to below about 0.100 ppm, preferably below 0.050 ppm, most preferably below 0.030 ppm.

As outlined above, preferred operation settings of the corona plasma device (s) in the said method are chosen in such a way to provide for:

- an electric field of about $10 \times 10^6$ V/m to about $20 \times 10^6$ V/m; and/or
- an energy density of about 400 $J/m^3$ to about 4,000 $J/m^3$; and/or
- a current density on the surface area of a field electrode within the range of about 0.4 $A/m^2$ to about 4.0 $A/m^2$.

Moreover, the said method is preferably carried out while assuring a transit time of the gaseous medium through the corona discharge device of about 30 ms to about 200 ms, preferably about 40 ms to about 175 ms, most preferably about 50 ms to about 150 ms, at a flow rate of about 0.5 m/s to about 16 m/s, preferably about 0.5 m/s to about 5 m/s, most preferably about 2 to about 3 m/s.

In yet another preferred embodiment of the said method, the flow of the gaseous medium at least through the treatment zone is controlled in such a way as to provide for an essentially non-turbulent flow. It has been found that a non-turbulent, preferably a laminar flow is highly preferred in comparison to the state of the art, where turbulences are described as preferred. In the present invention, precipitation of contaminants is desired, and turbulences would hamper reliable precipitation. In order to ensure uniformity of treatment across the air-stream and to additionally ensure evenness of ozone reduction activity across the surface of the downstream catalytic ozone mitigation device, it has been found that the velocity of the incident air-stream should be consistent across the width of the device. This can be most advantageously implemented by introducing a small pressure drop through a flow arresting device such as a filter or grill upstream of the treatment zone and ensuring that the incident airstream is as non-turbulent as possible.

The invention moreover relates to an HVAC system, comprising an air treatment device as outlined above and/or operating according to a method as outlined above.

Yet another aspect of the present invention relates to a disposable electrode, preferably a reference electrode, of a corona plasma device for use in an air treatment device as outlined above, wherein said electrode acts as the recipient for precipitation of contaminants, and where disposal of the electrode allows for the removal of said contaminants in a safe and straightforward fashion. Said electrode can be rendered still more effective by being made of or preferably essentially comprising a material with cytotoxic effect, preferably selected from the group consisting of copper, silver, or combinations or alloys thereof. Alternatively or additionally, the electrode may include a coating with a cytotoxic effect. Such coatings may include copper or silver ions, or common antimicrobial materials known to the person of routine skill in the art, e.g. based on organic acids and their salts, such as Potassium Sorbate. By using a disposable electrode, the full degree of operational reliability can be easily maintained.

According to the invention, a corona plasma electrode is used as a recipient for precipitation of contaminants of a gaseous medium conducted through a flow-through air treatment device, said air treatment device comprising a downstream ozone mitigation device, and prolonging the duration of said contaminant to reactive oxygen species, especially ozone ($0_3$), up to or exceeding an effective value necessary for decontamination.

Of course it is to be understood that other common aspects of plasma and/or ozone air treatment devices can be applied in the context of the present invention. For example, the person of routine skill in the art is *inter alia* familiar with the generation of other reactive oxygen species (e.g. the hydroxyl radical) from ozone by means of controlled humidity and e.g. UV radiation, what might be advantageously applied in the context of the present invention for certain contaminants.

The invention will now be described in more detail by means of specific examples and embodiments. The invention is, however, not to be limited to these aspects of the invention.

Fig. 1: overall arrangement of components in the air treatment device and test rig;

Fig. 2: schematic illustration of a corona discharge device with wire/tube geometry;

Fig. 3: schematic illustration of a corona discharge device with wire/plate geometry;

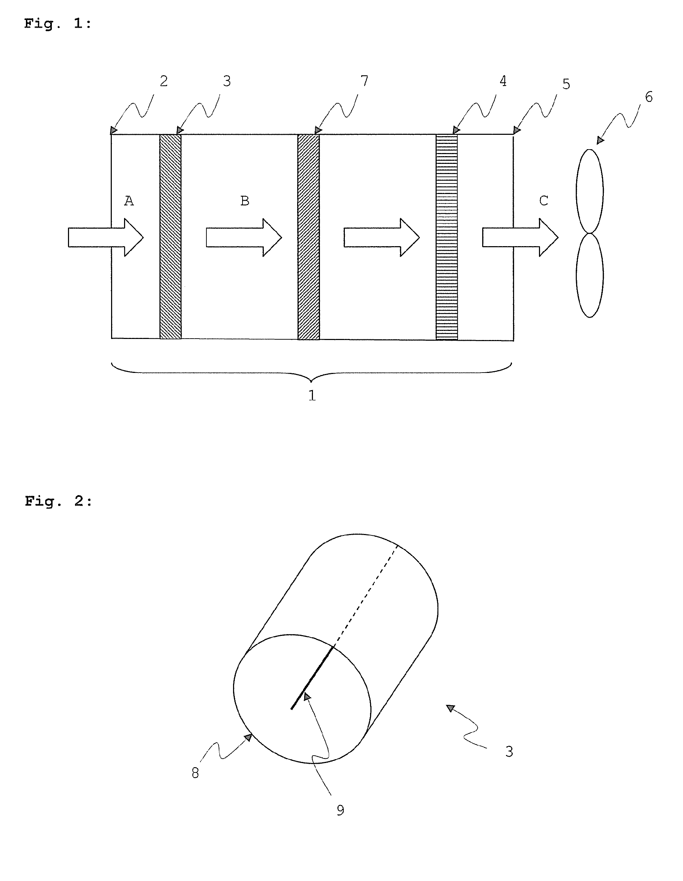

Fig. 1 illustrates an overall arrangement of the individual components in the air treatment device 1, and in the test rig used in the experiments discussed below. In Fig. 1, the flow of the medium (air) is directed from left to right, depicted by the arrows. A stream of contaminated air enters the air treatment device 1 via an inlet 2 and then reaches a corona discharge device 3. Prior to any contact with the corona discharge device, the degree of contamination in the medium is measured at a position A. Having passed the corona discharge device 3, the medium is passed through a particle filter 7, an ozone mitigation device 4 and finally leaves the air treatment device 1 via an outlet 5. The particle filter 7 protects the ozone mitigation device 4 from contaminations with particles that otherwise might hamper ozone depletion. The degree of contamination of the medium is analyzed in between the corona discharge device 3 and the ozone mitigation device 4 at position B, immediately upstream of the particle filter, and after the ozone mitigation device 4 at position C. The medium is sucked through the device by a suction device such as a pump or a fan 6.

Fig. 2 illustrates an embodiment of the corona discharge device 3 as used in the air treatment device 1 according to the invention. An outer reference electrode 8 of tube-like geometry is surrounding a central wire-like field electrode 9. As outlined above, a positive corona is preferred; the field electrode 9 is thus connected to the plus pole of a power supply, while the reference electrode 8 is connected to ground. For further electrical settings, it is referred to the description above. It is to be understood that variations especially of the geometry of the field electrode are possible within the context of the present invention, e.g. a star-like shape of the wire (in cross-sectional view) or the like could be used. Such variations are known to the person of routine skill in the art.

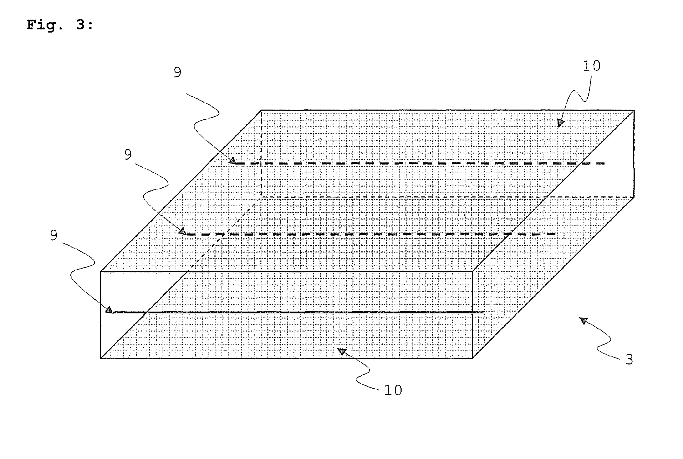

Fig. 3 illustrates another embodiment of the corona discharge device 3 as used in the air treatment device 1 according to the invention. The reference electrodes 10 are forming the inner walls of an essentially rectangular flow path. Again, wire-like field electrodes 9 are installed in the flow path, however, in this embodiment, they are installed approximately across the direction of flow of the medium to be treated. A plurality of field electrodes 9 are preferably installed in the flow path, thereby generating a plurality of plasma "curtains" to be passed by the medium during treatment.

In any embodiment, the electrodes, especially the reference electrodes are preferably designed as a disposable article, insertable and detachable from the corona discharge device if necessary due to e.g. contamination or abrasion.

An air treatment device has been thoroughly evaluated, comprising a corona discharge device with the following constructional and electrical details (see Fig 1 & 2):

- The corona discharge device (3), comprising two arrays of corona discharge electrodes; the arrays arranged in series, so that the incident airstream passes through them both.
  Each array consisting of 80 cylindrical reference electrodes (8)(internal diameter 24mm, length 200mm, material stainless steel), concentrically aligned about 80 field electrodes (9)(0.25 mm diameter, material stainless steel).

- High voltage power supply (not depicted) connecting the two electrode arrays in parallel. Power levels of up to 400W total provided at around 9.6kV.

- Downstream ozone mitigator module (4) comprising an activated carbon bed (manufactured by AAC Eurovent Ltd: AAC S/P, Model ref: DBF/12/25/10/60).

- In between the corona discharge device and the ozone mitigation device, a pre-filter has been used (7) (EN779-class G4, Cotton / Polyester blend pleated panel filter, 45mm x 600mm x 600mm, supplied by AAC Eurovent Ltd)

The performance of this air treatment device was determined at a flow rate of 318 $m^3$/h against various micro-organisms. This equates to a linear flowrate of around 2.5m/s through the corona discharge device. The bacterial tracers used were *Bacillus subtilis* var niger NCTC10073 (Gram positive, spore, 1.1 x 0.6 microns), washed vegetative cells of *Brevundimonas diminuta* NCIMB 11091 (Gram negative, rod, 0.3 x 0.8 microns) and vegetative cells of *Staphylococcus epidermidis* NCIMB 12721 (Gram positive, cocci, ca 0.6 microns diameter). *Bacillus subtilis* var niger spores are aerosol stable and are resistant to the action of many disinfectants, *Brevundimonas diminuta* is the smallest known free living micro-organism and *Staphylococcus epidermidis* is a non-pathogenic skin contaminant. The viral model used was MS-2 coliphage (NCIMB 10108), which is an unenveloped single stranded RNA coliphage, 23 nm in diameter with a molecular weight of 3.6 x $10^6$ Daltons. MS-2 coliphage sprayed from dilute nutrient broth is known to remain infectious under the conditions used here. When this suspension is sprayed from a Collison nebulizer, the airborne coliphage are carried in droplets, which are larger than the infectious particles, consisting mostly of media constituents.

Concentrations of the microorganism suspensions used in the present experiments were as follows:

*Bacillus subtilis* var niger: 3.20 x $10^9$ cfu per ml
*Brevundimonas diminuta:* 4.60 x $10^{10}$ cfu per ml
*Staphylococcus epidermidis:* 6.00 x $10^9$ cfu per ml
MS-2 coliphage: 3.65 x $10^{11}$ pfu per ml

Test rig for challenging the AP Unit with airborne micro-organisms

The test rig is shown schematically in Figure 1. Filtered air (using a coarse filter) was drawn through the rig at a flow rate of 318 $m^3$ $hr^{-1}$ by a fan unit. The air was initially drawn into a spray chamber housing a 6-jet Collison spray.

The Collison spray contained a suspension (10-20 ml) of the appropriate test micro-organism. The microbial suspension was aerosolised by applying compressed air to the Collison spray at 180 KPa for 10 minutes. The breakthrough and challenge were sampled simultaneously, during the 10 minutes, via the 3 test ports provided as illustrated, one upstream (Position A), one downstream (Position C) and one between the corona discharge device and the ozone mitigation device (Position B). The aerosolised microbes were passed into a mixing chamber and then into a duct which housed the corona discharge device and the ozone mitigation device. At the downstream end of the housing, another length of duct was connected which is linked to the fan units via a back-up HEPA filter assembly which was designed to prevent the escape of any remaining airborne micro-organisms into the environment.

Microbiological air sampling

Cyclone samplers were used to sample the air via the three test ports in the ducting described above. The clean glass cyclone samplers were linked to a vacuum pump so that approximately 700 litres of air per minute could be sampled both upstream and downstream of the air treatment device and between the corona discharge device and the ozone mitigation device. The collecting fluid (Phosphate buffer plus manucol and antifoam) (PBMA) was injected into the inlet of the cyclone at a rate of about 1-2 ml per minute. The airborne particles containing the micro-organisms were deposited by centrifugal forces on the cyclone wall and washed off by the swirling collection fluid. The fluid was withdrawn from the base of the cyclone by a syringe at the end of the sampling period. The volume of fluid collected was measured.

Test Methodology

1. The corona discharge device and the ozone mitigation device to be tested were placed into the test rig using the appropriate adapters, gaskets and bolts to prevent the leakage of air.
2. The air flow was adjusted to the required volume (318 $m^3$ $hr^{-1}$) by switching on the fans and fine adjusting the flow rate by opening or closing the iris in the duct. The mean flow rate was determined using the anemometer.
3. The sterile cyclone samplers were fitted into the ports upstream (Position A) and downstream (Position C) of the corona discharge device and one in between the corona discharge device and the ozone mitigation device (Position B). The three vacuum pumps were set to sample 600-800 litres $min^{-1}$ air and the three peristaltic pumps were set to inject PBMA collecting fluid from the bottle to the cyclone inlet at approximately 1.5 ml per minute.
4. A background sample was taken from the rig using the cyclone samplers (B + C) for 10 minutes. The collecting fluid was removed at the bottom of the cyclones using a syringe. The samples were transferred to pre-weighed sterile universal containers which were then re-weighed to give the exact volume collected.
5. Preliminary studies were originally carried out using B. *subtilis* var niger to determine the performance of the corona discharge device with and without the ozone mitigation device (ports C + B, respectively) being used. The bacterial suspension of *B. subtilis* var niger (20-50 ml) was pipetted into the Collison nebuliser and the Collison nebuliser was then placed in the spray chamber.
6. The Collison nebuliser was activated by attaching it to the compressed air source at 180 Kpa and at the same time switching on the three cyclone samplers (A, B + C). The nebuliser was switched off after 10 minutes (the cyclone samplers were switched off 15 seconds after the nebuliser).
7. The collecting fluid from the three cyclone samplers was removed using the syringes as described in 4 and the volume was determined by weighing the pre-weighed sterile universal containers.
8. The nebuliser was refilled, if necessary, with bacterial suspension and procedure numbers 6 and 7 were repeated 3 times with the corona discharge device turned off and 3 times with the corona discharge device switched on.
9. The collecting fluid from each of the three cyclone samplers (A, B + C) was assayed using standard microbial culturing assay techniques as described in the test micro-organisms section above.
10. The total number of micro-organisms challenging the corona discharge device per $m^3$ of air was determined from the colony forming units assayed in the A cyclone sample as follows:

$$X = \frac{(\text{cfu per } 0.1 \text{ ml}) \times (\text{dilution}) \times (\text{total volume obtained})}{(\text{volume of air sampled})}$$

11. The total number of micro-organisms collected downstream of either the corona discharge device plus the ozone mitigation device (C) or just the corona discharge device (B) per $m^3$ of air was determined from the colony forming units assayed in B + C cyclone samples as above.

12. At the end of one of the runs with the corona discharge device turned off and one of the runs with AP Unit switched on the front and back faces of the corona discharge device was swabbed in 6 places with a wetted sterile swabs. Each of the 6 swabs was carried out by removing the swab from its sterile container, wetting with sterile distilled water and swabbing approximately a $cm^2$ area of the entrance of one of the tubes. The swab was then streaked onto a TSBA plate.

13. Six tests were also carried out to observe if the ozone generated by the corona discharge device had a biocidal effect on the *B. subtilis.* These tests were carried out by running the rig as described above except the nebuliser was not activated and only cyclone B was switched on. The PBMA added to cyclone B was spiked with a known concentration of the B. *subtilis.* This test was carried out three times with the corona discharge device on and three times with the corona discharge device off.

14. Using all of the results obtained from the tests described above, the bacterial performance of the air treatment device against *B. subtilis* was determined.

15. The test methodology described above (1-14) repeated for each of the other three organisms:

> *Brevundimonas diminuta* NCIMB 11091;
> *Staphylococcus epidermidis* NCIMB12721;
> MS2 coliphage.

16. The six downstream samples (port B) generated from the *Brevundimonas diminuta* runs were assayed to determine the endotoxin levels present.

Calculation of Percentage Efficiency

The percentage efficiency of the AP Unit for each of the micro-organisms was calculated using the following formula where A is the total number of colony forming units per cubic meter of air ($cfu/m^3$) or plaque forming units per cubic meter of air ($pfu/m^3$) challenging the unit and B is the total number of $cfu/m^3$ or $pfu/m^3$ exiting the unit:

$$\texttt{\% efficiency} = \frac{A - B}{B} \texttt{ x 100}$$

Results

The results of the tests are summarised below as average % efficiency of the system at position B and C with the device switched on and off, respectively:

| Microorganism | Device on / off | Position B | Position C |
|---|---|---|---|
| Bacillus subtilis var niger | On | 99.75 | 99.973 |
| Bacillus subtilis var niger | Off | (-8.24) | 28.62 |
| Brevundimonas diminuta | On | 99.89 | 99.9979 |
| Brevundimonas diminuta | Off | (-15.84) | (-12.03) |
| Staphylococcus epidermidis | On | 99.77 | 99.969 |
| Staphylococcus epidermidis | Off | (-35.6) | 19.0 |
| MS-2 coliphage | On | 99.9907 | 99.9933 |
| MS-2 coliphage | Off | (-34.82) | 20.64 |

Endotoxin results with *Brevundimonas diminuta* runs exhibited an average reduction ($EU/m^3$) of 99.89% (corona discharge device on/off, measured three times each).

The air treatment device reduces the levels of the all the micro-organisms by at least 99.75%. The ozone eliminator placed after the air purification device caused at least a further 90% loss in the airborne concentration of all the bacteria. However, the average loss on the case of the phage caused by the ozone eliminator was lower.

None of the bacteria were affected by the ozone in the spiking experiments with no significant loss of viability detected in these tests for all the bacteria. The swabbing results showed that operation of the device reduced the levels of all of the bacteria recovered from surfaces compared to those found with the machine switched off. In some occasions slightly higher concentrations of the airborne micro-organisms were found at position B and this is indicated as slightly negative reductions when the device was switched off. This is either due to slightly higher efficiency of collection of the cyclone at position B, a slightly higher collection rate, a slightly different geometry of the collection port or a combination of these factors.

The viral results obtained with MS-2 phage were more complex. The spiking experiments indicated that ozone could reduce the numbers of cfu in the collection fluid of cyclone B to below the detection limit. However, when the main experiment was carried out phage were recovered from position B and a reduction in concentration of 99.9907% was measured. The reduction after the ozone eliminator amounted to 99.9933%. The reduction caused by the ozone eliminator was less than that found with all the bacteria which could be due to the smaller size of the virus, although the particle sizes should be similar to those of the bacteria. A possible explanation for all the phage results may be that ozone can inflict two forms of damage on the phage. The first action irreversibly inactivates the virus while the second mode of action causes reversible damage which can be repaired by physical means. If the phage collected by the cyclone is assayed soon after sampling then the reversible damage may not have been repaired but after a longer storage period the initial damage may be reversed. However, these experiments indicate that ozone is more active against the MS-2 phage than all the bacteria tested.

In order to evaluate which amount of micro-organisms might already be killed due to exposure just to the gases generated by the cold plasma, i.e. without precipitation of the contaminants on the electrode(s), the inlet air stream was split into two streams, with only one of the two streams passing through the plasma device. Subsequently, prior to the ozone mitigation device, the two streams of air are recombined in a mixing chamber. Two performance tests (with the plasma device switched on) and two baselines (with the plasma device switched off) were conducted. In the first set of tests, yeast was nebulised into the air stream that bypasses the plasma device. In the second set of tests, the yeast was nebulised into the air stream that passes through the plasma device. Two Biotrace Air Samplers were used (Air Trace Environmental Air Sampler; Product Code: ATEM240, Biotrace Fred Baker Ltd): one sampling air before reaching either the plasma device or the mixing chamber, and the other sampling air leaving the mixing chamber. Microbiological tests were conducted as outlined above. The set-up was as follows:

- The plasma device comprised a single array of 80 tubes (wire to cylinder geometry, as outlined above);
- Each tube of the array was 24 mm ID, 200 mm long, stainless steel 316;
- Wire was 0.25 mm diameter, stainless steel 302;
- Power was approximately 94 - 98W, 7.2kV negative DC;
- Air speed was approximately 1.4 m/s in each inlet stream, and 1.2 m/s through the tubes of the plasma device;
- Air speed was set to approximately 250 - 280 m$^3$/hr);
- Diameter of inlet ducts and outlet ducts = 200 mm;
- Maximum internal dimensions of mixing chamber = 380H x 380D x 680W, and overall length with transition pieces attached at either end was approximately 1m.
- Separation of inlet streams approximately 350 mm between centrelines.

In the first instance, the tests were performed with the plasma device switched off to understand the percentage of microorganisms lost in the system. Since these results show a significant loss of yeast cells due to the set-up of the system, a correcting factor was used to calculate the efficiency of the plasma device itself. The additional removal achieved when the AP is on during a test run, can be calculated as:

$$\frac{A \times (100 - \text{loss in baseline}) - B}{A \times (100 - \text{loss in baseline})} \times 100$$

(A: nebulizer position in stream with plasma device; B: nebulizer position in stream bypassing the plasma device).

Results corrected for the baselining factor from the tests with the AP on as average % efficiency of the system at the downstream sampler are shown in the table below.

| Nebuliser position | Unit on/off | Downstream sampler - average reduction |
|---|---|---|
| nB (with AP) | on | 99.947% |
| nA (without AP) | on | 78.420% |

Thus, an additional reduction (>log3) is achieved with the plasma device switched on, when nebulising into the duct containing the plasma device.

The results indicate that a significant part of microorganism removal is achieved in the gas phase (as with the prior art devices mentioned in the introductory part), most likely through the actions of gases produced by the AP such as $O_3$, $NO_X$ and others.

However, when organisms are directly passed through the plasma device, this gas phase kill is aided inter alia by precipitation of the organisms onto the surfaces of the electrode(s) and subsequent killing through the prolonged exposure to the actions of the AP.

**Claims**

1. Flow-through air treatment device (1), comprising

   i) an inlet (2) for a gaseous medium to be treated,
   ii) an air treatment zone, comprising a corona discharge device (3) located in said air treatment zone, which corona discharge device in use creates ozone ($O_3$) at a concentration of at least 1 ppm, preferably about 1 ppm to about 50 ppm, most preferably about 2 ppm about 30 ppm;
   iii) a downstream ozone mitigation device (4), allowing for a reduction of the ozone ($O_3$) concentration to below about 0.100 ppm, preferably about 0.075 ppm, most preferably about 0.050 ppm;
   iv) an outlet (5) for the treated gaseous medium.

2. Air treatment device according to claim 1, wherein the corona discharge device (3) generates an electric field of about $10 \times 10^6$ V/m to about $20 \times 10^6$ V/m.

3. Air treatment device according to one of claims 1 or 2, wherein the corona discharge device (3) allows for establishing of a positive corona plasma.

4. Air treatment device according to claim 3, wherein the corona discharge device (3) is supplied with a pulsed DC.

5. Air treatment device according to one of claims 1 to 4, wherein a suction device, preferably a fan (6) or a pump, is arranged downstream of the ozone mitigation device.

6. Air treatment device according to one of claims 1 to 5, wherein the suction device is operable in such a way to allow for the gaseous medium being conducted through the treatment zone at a flow rate of about 0.5 m/s to about 16 m/s, preferably about 0.5 m/s to about 5 m/s, most preferably about 2 to about 3 m/s.

7. Air treatment device especially according to one of claims 1 to 6, comprising a corona discharge device (3), wherein an electrode, preferably the reference electrode of said corona discharge device is made of or preferably essentially comprises a material with cytotoxic effect, preferably selected from the group consisting of copper, silver, or combinations or alloys thereof, and/or includes a coating with cytotoxic effect, preferably chosen from the group consisting of coatings comprising silver ions, copper ions, organic acids and their salts, and combinations thereof.

8. Air treatment device according to one of claims 1 to 7, wherein an energy density of about 400 J/m$^3$ to about 4,000 J/m$^3$ is created in the treatment zone.

9. Air treatment device according to one of claims 1 to 8, wherein the current density on the surface area of a field electrode is within the range of about 0.4 A/m$^2$ to about 4.0 A/m$^2$ .

10. Air treatment device according to one of claims 1 to 9, wherein, at flow rates of about 0.5 m/s to about 16 m/s, preferably about 0.5 m/s to about 5 m/s, most preferably about 2 to about 3 m/s, the corona discharge device (3) is designed in such a way to allow for a transit time of the gaseous medium through the corona discharge device (3) of about 30 ms to about 200 ms, preferably about 40 ms to about 175 ms, most preferably about 50 ms to about 150 ms.

11. Air treatment device according to one of claims 1 to 10, wherein the corona discharge device (3) is operable in such a way to allow for a precipitation of contaminants from the gaseous medium on an electrode, preferably the reference electrode.

**12.** Air treatment device according to one of claims 1 to 11, wherein the treatment zone comprises at least one, preferably a multitude of corona discharge device(s) (3), comprising an outer reference electrode (8) of tube-like geometry as a flow-through passage for said gaseous medium to be treated, said reference electrode (8) surrounding an inner field electrode (9) of wire-like geometry.

**13.** Air treatment device according to claim 12, wherein the field electrode(s) is/are arranged essentially in parallel with the direction of the flow of the gaseous medium.

**14.** Air treatment device especially according to one of claims 1 to 11, wherein the treatment zone comprises at least one, preferably a multitude of corona discharge device(s) (3), comprising at least one reference electrode (10) of plate-like geometry, and a field electrode (9) of wire-like geometry, preferably two plate-like reference electrodes (10) arranged on opposite sides of the wire-like field electrode.

**15.** Air treatment device according to claim 14, wherein the field electrode(s) (9) is/are arranged essentially perpendicular to the direction of the flow of the gaseous medium.

**16.** Air treatment device according to one of claims 14 or 15, wherein the plate-like electrode(s) (10) is/are wall(s) of a flow-through passage for said gaseous medium to be treated.

**17.** Air treatment device according to one of claims 1 to 16, comprising a flow-meter and/or an ozone ($O_3$) sensor, within the treatment zone and/or at the outlet.

**18.** Air treatment device according to one of claims 1 to 17, wherein the ozone mitigation device (4) comprises a preferably disposable catalytic ozone mitigator, preferably comprising a zeolith with an $SiO_2/Al_2O_3$ ratio of not less than 15, and activated manganese dioxide ($MnO_2$).

**19.** Air treatment device according to one of claims 1 to 18, further comprising a preferably disposable particle filter (7) upstream of the ozone mitigation device (4).

**20.** A method of treating a gaseous medium, comprising the steps of:

i) preferably continuously conducting said gaseous medium through a treatment zone, said treatment zone comprising at least one preferably positive corona discharge device (4) creating ozone ($O_3$) at a concentration of at least 1 ppm, preferably about 1 ppm to about 50 ppm, most preferably about 2 ppm to about 20 ppm;
ii) precipitating contaminants from the gaseous medium on an electrode, preferably the reference electrode(s) (9,10) of said corona plasma device (4);
iii) conducting said gaseous medium through a downstream ozone mitigation device (4), allowing for a reduction of the ozone ($O_3$) concentration to below about 0.100 ppm, preferably below 0.050 ppm, most preferably below 0.030 ppm.

**21.** A method according to claim 20, wherein the operation settings of the corona plasma device(s) (4) are chosen in such a way as to provide for:

- an electric field of about $10 \times 10^6$ V/m to about $20 \times 10^6$ V/m; and/or
- an energy density of about 400 J/m$^3$ to about 4,000 J/m$^3$; and/or
- a current density on the surface area of a field electrode within the range of about 0.4 A/m$^2$ to about 4.0 A/m$^2$.

**22.** A method according to one of claims 20 or 21, wherein the transit time of the gaseous medium through the corona discharge device (4) is about 30 ms to about 200 ms, preferably about 40 ms to about 175 ms, most preferably about 50 ms to about 150 ms, at a flow rate of about 0.5 m/s to about 16 m/s, preferably about 0.5 m/s to about 5 m/s, most preferably about 2 to about 3 m/s.

**23.** A method according to one of claims 20 to 22, wherein the flow of the gaseous medium at least through the treatment zone is controlled in such a way as to provide for an essentially non-turbulent flow.

**24.** A method according to one of claims 20 to 23, wherein the treatment is carried out by an air treatment device (1) according to one of claims 1 to 19.

**25.** An HVAC system, comprising an air treatment device (1) according to one of claims 1 to 19 and/or operating according to a method of one of claims 20 to 24.

**26.** A disposable electrode, especially a reference electrode (9,10) of a corona plasma device (4) for use in an air treatment device (1) according to one of claims 1 to 19.

**27.** A disposable electrode according to claim 26, wherein said electrode is made of or preferably essentially comprises a material with cytotoxic effect, preferably selected from the group consisting of copper, silver, or combinations or alloys thereof, and/or includes a coating with cytotoxic effect, preferably chosen from the group consisting of coatings comprising silver ions, copper ions, organic acids and their salts, and combinations thereof.

**28.** Use of a corona plasma electrode as a recipient for precipitation of contaminants of a gaseous medium conducted through a flow-through air treatment device (1), said air treatment device (1) comprising a downstream ozone mitigation device (4), and thereby prolonging the duration of exposure of said contaminant to reactive oxygen species, especially ozone ($O_3$), up to or exceeding an effective value necessary for decontamination.

Fig. 1:

Fig. 2:

EP 1 867 346 A1

Fig. 3:

**European Patent Office**

## EUROPEAN SEARCH REPORT

Application Number

EP 06 11 5390

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 055 115 A (YIKAI KUNIO [JP] ET AL) 8 October 1991 (1991-10-08) | 1,3,5, 11-13, 19,20, 24,25,28 | INV. A61L9/015 F24F3/16 A61L9/22 |
| Y | * column 2, line 21 - column 4, line 24 * * column 5, line 33 - column 6, line 21; figure 2 * | 7 | |
| X | US 5 814 135 A (WEINBERG STANLEY [US]) 29 September 1998 (1998-09-29) | 1,3,5, 11, 18-20, 24,28 | |
| | * column 3, line 26 - column 5, line 42; figure 2 * | | |
| Y | EP 1 434 014 A2 (SAMSUNG ELECTRONICS CO LTD [KR]) 30 June 2004 (2004-06-30) * paragraphs [0030] - [0032], [0034], [0037] - [0039], [0041], [0045], [0049]; figure 3A * | 7 | |
| X | DE 199 03 022 A1 (SEISUI SENDAI KK [JP]) 28 October 1999 (1999-10-28) | 1,2, 10-13, 18,20, 24,25 | TECHNICAL FIELDS SEARCHED (IPC) F24F A61L |
| | * column 3, line 10 - column 4, line 31 * * column 5, line 4 - line 16 * * column 6, line 25 - line 54 * | | |

~~The present search report has been drawn up for all claims~~

| Place of search | Date of completion of the search | Examiner |
|---|---|---|
| Munich | 14 November 2006 | KATSOULAS, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent

Office

**Application Number**

EP 06 11 5390

---

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-25, 28

16

**European Patent Office**

**LACK OF UNITY OF INVENTION**
**SHEET B**

Application Number

EP 06 11 5390

The Search Division considers that the present European patent application does not comply with the requirements of unity of invention and relates to several inventions or groups of inventions, namely:

1. claims: 1-25,28

    Independent claims 1 and 20 concern an air treatment device and its method of operation, the device compising (a) an air treatment zone comprising an ozone producing corona discharge device, (b) a downstream ozone mitigation device and (c) an inlet and outlet for air.
    Independent claim 25 defines a HVAC system comprising the device of claim 1 and/or operating according to method claim 20.
    Independent claim 28 defines a use of a corona plasma electrode as a recipient for precipitation of contaminants in an air treatment device comprising a downstream ozone mitigation device.
                            ---

2. claims: 26, 27

    Independent claim 26 defines a disposable electrode of a corona plasma device suitable of being used in an air treatment device of claim 1.
                            ---

## ANNEX TO THE EUROPEAN SEARCH REPORT
## ON EUROPEAN PATENT APPLICATION NO.

EP 06 11 5390

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2006

| Patent document cited in search report | | Publication date | Patent family member(s) | | Publication date |
|---|---|---|---|---|---|
| US 5055115 | A | 08-10-1991 | DE | 3921387 A1 | 05-07-1990 |
| | | | JP | 2172545 A | 04-07-1990 |
| US 5814135 | A | 29-09-1998 | EP | 0824041 A2 | 18-02-1998 |
| | | | JP | 10180139 A | 07-07-1998 |
| | | | US | 5667564 A | 16-09-1997 |
| EP 1434014 | A2 | 30-06-2004 | CN | 1510357 A | 07-07-2004 |
| | | | KR | 20040056133 A | 30-06-2004 |
| | | | US | 2004129140 A1 | 08-07-2004 |
| DE 19903022 | A1 | 28-10-1999 | JP | 3046951 B2 | 29-05-2000 |
| | | | JP | 11300151 A | 02-11-1999 |
| | | | US | 6508982 B1 | 21-01-2003 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2413377 A
- WO 03028773 A1
- WO 0033945 A